# FASCICULE DE BREVET EUROPEEN

(11) **EP 1 902 754 B1**
(45) Date de publication et mention de la délivrance du brevet: **29.04.2009**
(21) Numéro de dépôt: 07115311.8
(22) Date de dépôt: 30.08.2007
(51) Int. Cl.: A61Q 15/00, A61K 8/06, A61K 8/27, A61K 8/34, A61K 8/365, A61K 8/368

(54) **Composition déodorante hydroalcoolique comprenant un sel hydrosoluble de zinc et l'acide salicylique**
Hydroalkoholische Deodorantzusammensetzung, die ein wasserlösliches Zinksalz und Salicylsäure umfasst
Hydro-alcoholic deodorant composition containing a water-soluble salt of zinc and salicylic acid

(30) Priorité: 19.09.2006 FR 0653810
(43) Date de publication de la demande: 26.03.2008
(73) Titulaire: L'Oréal, 75008 Paris (FR)
(72) Inventeur: PRUD'HOMME, Estelle, 75018, PARIS (FR)
(74) Mandataire: Miszputen, Laurent

(56) Documents cités:
- EP-A- 0 714 655
- EP-A- 1 486 199
- EP-A1- 0 768 080
- WO-A-00/30601
- WO-A-00/32242
- US-B1- 6 426 061

## Description

La présente invention concerne une composition déodorante comprenant dans un milieu cosmétiquement acceptable
a) au moins un sel hydrosoluble de zinc,
b) l'acide salicylique
c) au moins un monoalcool en C₁-C₅ ;
d) au moins 2% en poids d'eau par rapport au poids total de la composition.

Elle concerne également un procédé cosmétique pour traiter les odeurs axillaires humaines consistant à appliquer sur la surface axillaire une quantité efficace de ladite composition.

Dans le domaine cosmétique, il est bien connu d'utiliser en application topique, des produits déodorants contenant des substances actives de type anti-transpirant ou de type bactéricide pour diminuer voire supprimer les odeurs axillaires généralement désagréables.

Les substances bactéricides détruisent en général la flore bactérienne résidente. Parmi ces substances, les plus employées sont le Triclosan (2,4,4'-trichloro-2'-hydroxydiphényléther) et le farnésol qui présentent l'inconvénient de modifier de façon importante l'écologie de la flore cutanée. Il y a les substances qui diminuent la croissance des bactéries. Parmi ces substances, on peut citer les chélatants de métaux de transition comme l'EDTA ou le DPTA. Ces matériaux privent le milieu des métaux nécessaires à la croissance des bactéries. Ces actifs sont malheureusement potentiellement écotoxiques et peuvent causer des problèmes d'environnement.

Les substances anti-transpirantes ont pour effet de limiter le flux sudoral. Elles sont généralement constituées de sels d'aluminium qui d'une part sont irritants pour la peau et qui d'autre part diminuent le flux sudoral en modifiant la physiologie cutanée, ce qui n'est pas satisfaisant.

Dans cette optique, à titre d'actif déodorant, certains sels hydrosolubles de zinc ont déjà été proposés dans des produits déodorants notamment sous forme d'émulsion eau-dans-silicone comme par exemple le pyrrolidone carboxylate de zinc (plus communément appelé pidolate de zinc), le sulfate de zinc, le chlorure de zinc, le lactate de zinc, le gluconate de zinc et le phénolsulfonate de zinc. De telles formulations déodorantes ont été notamment décrites dans les documents WO93/01793, EP468564, EP024176, EP768080, EP1486199. Ces sels hydrosoluble de zinc ont un spectre bactéricide très restreint, et en particulier une efficacité déodorante non liée à une activité bactéricide sur les germes responsables des mauvaises odeurs. Lorsqu'ils sont formulés en milieu hydroalcoolique, après application sur la peau et évaporation des solvants, il ne reste donc sur la peau que des composés n'ayant pas d'activité bactéricide, donc ne risquant pas d'entraîner une résistance aux bactéries. Cependant, ces formulations hydro-alcooliques à base de ces sels hydrosolubles de zinc ont tendance à se déstabiliser et à former des composés basiques insolubles qui précipitent et donne un aspect inesthétique.

La demande de brevet WO00/038644 décrit également des produits déodorants aqueux pouvant contenir de l'alcool comprenant l'association d'un sel de zinc et d'un sel de bicarbonate ou carbonate stabilisée par la présence d'anions issus de di-, tri ou polyacides ou de di-, tri ou poly-phosphates. Ces anions polyacides ou polyphosphates en complexant l'ion zinc ont l'inconvébient de diminuer fortement son efficacité déodorante.

Il subsiste le besoin de trouver de nouvelles formulations hydro-alcooliques à base de sel de zinc hydrosoluble qui soient efficaces en activité déodorante sans les inconvénients des formulations de l'art antérieur.

La demanderesse a découvert de manière surprenante qu'en ajoutant l'acide salicylique dans une formulation hydroalcoolique à base de sel de zinc hydrosoluble, on obtenait une composition stable sans formation de précipité, ayant une bonne efficacité déodorante non liée à une activité bactéricide.

Cette découverte est la base de la présente invention.

La présente invention a ainsi pour objet une composition déodorante comprenant dans un milieu cosmétiquement acceptable :
a) au moins un sel hydrosoluble de zinc
b) l'acide salicylique,
c) au moins un monoalcool en C₁-C₅ et
d) au moins 2% en poids d'eau par rapport au poids total de la composition.

On entend au sens de la présente invention par « composition déodorante », toute composition capable de produire une diminution des mauvaises odeurs liées à la dégradation de la sueur

La présente invention a également pour objet l'utilisation de l'acide salicylique dans une composition déodorante comprenant dans un milieu cosmétiquement acceptable :
(a) au moins un sel hydrosoluble de zinc
(b) au moins 2% en poids d'eau par rapport au poids total de la composition,
(c) au moins un monoalcool en C₁-C₅
comme agent stabilisant de la composition.

Elle a enfin pour objet un procédé de désodorisation mettant en oeuvre ladite composition, et plus particulièrement un procédé pour traiter les odeurs axillaires humaines, consistant à appliquer sur la surface axillaire une quantité efficace de ladite composition.

Au sens de la présente invention, on entend par « sel hydrosoluble de zinc », tout sel qui, après avoir été complètement dissous sous agitation à 1% dans une solution d'eau à une température de 25°C, conduit à une solution comprenant une quantité de sel insoluble inférieure à 0,05% en poids.

Parmi les sels hydrosolubles de zinc utilisables selon la présente invention, on peut citer le pyrrolidone carboxylate de zinc (plus communément appelé pidolate de zinc), le sulfate de zinc, le chlorure de zinc, le lactate de zinc, le gluconate de zinc, le phénolsulfonate de zinc, le salicylate de zinc et ses dérivés ou leurs mélanges.

Le salicylate de zinc et ses dérivés selon l'invention répondent en général à la structure suivante : 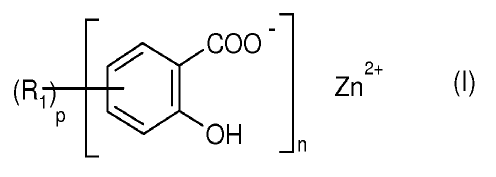 dans laquelle n = 2, p vaut 0, 1, 2 ou 3 ; R₁ désigne un alkyle en C₁-C₁₈ linéaire ou ramifié (par exemple méthyle, éthyle, n-propyle, isopropyle, n-butyle); un radical hydroxyalkyle en C₁-C₁₈, linéaire ou ramifié ; un atome d'halogène (par exemple Iode, Brome, Chlore) ; un radical acyle en C₂-C₁₈ (par exemple acétyle) ; un radical COR₂, OCOR₂ ou CONHR₂ où R₂ désigne un atome d'hydrogène ou un radical alkyle en C₁-C₁₈ linéaire ou ramifié.

On choisira plus préférentiellement le salicylate de zinc (p = 0) et plus particulièrement sous forme hydratée comme le salicylate de zinc trihydraté tel que le produit commercial vendu sous le "Zinc Salicylate Trihydrate" par la société Bernardy Chimie.

Dans les compositions déodorantes selon la présente invention, le sel hydrosoluble de zinc est généralement présent dans des proportions pondérales, allant de préférence de 0,1 à 5%, et mieux encore allant de 0,1 à 2%, par rapport au poids total de la composition.

L'acide salicylique est de préférence présent dans des proportions pondérales, allant de préférence de 0,01 à 0,5% et plus particulièrement de 0,05 à 0,2% en poids par rapport au poids total de la composition.

Les compositions déodorantes de la présente invention sont formulées classiquement selon les applications auxquelles elles sont destinées.

Le ou les mono-alcools en C₁-C₅ présents dans les compositions de l'invention peuvent être choisis parmi le méthanol, l'éthanol, l'isopropanol ou leurs mélanges. On choisira plus particulièrement l'éthanol. Ils sont de préférence présents à des concentrations supérieures à 10% en poids et plus préférentiellement allant de 15 à 96 % en poids par rapport au poids total de la composition.

La quantité d'eau est de préférence de 2 à 85%, en poids et plus préférentiellement de 2 à 30% en poids par rapport au poids total de la composition.

Les compositions déodorantes selon l'invention destinées à l'usage cosmétique peuvent se présenter sous forme de lotions distribuées en vaporisateurs ou pompe aérosol , de crèmes distribuées en tube ou en grille ; de gels distribués en roll-on ou en grille ; et contenir à cet égard les ingrédients généralement utilisés dans ce type de produits et bien connus de l'homme de l'art, sous réserve qu'ils n'interfèrent pas avec le sel de zinc hydrosoluble et le monoacide carboxylique décrits dans la présente invention.

Les compositions déodorantes selon l'invention destinées à l'usage cosmétique peuvent se présenter sous forme d'émulsion comme par exemple une émulsion eau-dans-huile, une émulsion huile-dans-eau, une émulsion multiple (émulsion triple huile-dans-eau-dans-huile ou eau-dans-huile-dans-eau (de telles émulsions sont connues et décrites par exemple par C. FOX dans « Cosmetics and Toiletries » - november 1986 - Vol 101 - pages 101-112).

Les compositions selon l'invention peuvent contenir en plus une phase liquide organique non-miscible dans l'eau. Celle-ci comprend en général un ou plusieurs composés hydrophobes qui rendent ladite phase non-miscible dans l'eau. Ladite phase est liquide (en l'absence d'agent structurant) à température ambiante (20-25 °C). De manière préférentielle, la phase liquide organique non-miscible dans l'eau conforme à l'invention est généralement constituée d'une huile ou de mélange d'huiles et comprend au moins 80% de composés ayant une vapeur de pression ne dépassant pas la valeur 4 kPa (30 mm Hg) à 25°C.

La phase liquide organique non-miscible dans l'eau contient de préférence une ou plusieurs huiles émollientes siliconée ou hydrocarbonée, volatiles ou non-volatiles. Ces huiles émollientes sont notamment décrites dans les brevets US 4,822,596 et US 4,904,463.

Les silicones volatiles sont définies de façon connue comme des composés volatils à température ambiante. On peut citer parmi ces composés les silicones volatiles cycliques et linéaires du type diméthylsiloxane dont les chaines comprennent de 3 à 9 résidus siliconés. De préférence on choisit les cyclométhicones D4, D5 ou D6.

Les silicones non-volatiles sont définies de façon connue comme des composés de pression de vapeur faible à température ambiante. Parmi ces composés sont inclus : les polyalkylsiloxanes, en particulier les polyalkylsiloxanes linéaires comme par exemple les polydiméthylsiloxanes, ou diméthicones, linéaires, commercialisés par la société Dow Corning sous le nom de « Dow Corning 245 Fluid » ; les polyalkylarylsiloxanes comme par exemple les polyméthylphénylsiloxanes, commercialisés par la société Dow Corning sous le nom de « Dow Corning 556 Fluid » ; les copolymères polyéther et siloxane, comme par exemple les diméthicone copolyols.

Parmi les huiles émollientes non-volatiles utilisables dans la présente invention, on peut citer par exemple : les dérivés hydrocarbonés, les huiles minérales, les alcools gras, les esters d'alcools en C₃-C₁₈ avec des acides en C3-C18, les esters de l'acide benzoïque avec des alcools en C₁₂-C₁₈ et leurs mélanges, des polyols en C₂-C₆ choisis de préférence parmi le glycérol, le propylèneglycol ou le sorbitol, les polymères de polalkylène glycol.

Les huiles émollientes sont présentes de préférence dans des quantités allant de 1 à 50% en poids et plus préférentiellement de 5 à 40% en poids par rapport au poids total de la composition.

La composition cosmétique déodorante selon l'invention peut contenir un ou plusieurs actifs déodorants additionnels comme par exemple des agents bactériostatiques ou des agents bactéricides tels que le 2,4,4'-trichloro-2'-hydroxydiphényléther (Triclosan), le 2,4-dichloro-2'-hydroxydiphényléther, le 3',4',5'-trichlorosalicylanilide, la 1-(3',4'-dichlorophenyl)-3-(4'-chlorophenyl)urée (Triclocarban) ou le 3,7,11-triméthyldodéca-2,5,10-triénol (Farnesol) ; les sels d'ammonium quaternaires comme les sels de cetyltrimethylammonium, les sels de cétylpyridinium ; la chlorhexidine et les sels; le monocaprate de diglycérol, le monolaurate de diglycérol , monolaurate de glycérol ; les sels de polyhexaméthylène biguanide.

La composition cosmétique déodorante selon l'invention peut contenir un ou plusieurs sels d'aluminium antitranspirants.

Par " sel d'aluminium anti-transpirant", on entend tout sel ou tout complexe d'aluminium ayant pour effet de diminuer ou limiter le flux sudoral.

Les sels d'aluminium conformes à l'invention sont choisis de préférence parmi les halohydrates de d'aluminium ; les halohydrates d'aluminium et de zirconium, les complexes d'hydroxychlorure de zirconium et d'hydroxychlorure d'aluminium avec ou sans un acide aminé tels que ceux décrits dans le brevet US-3792068.

Parmi les sels d'aluminium, on peut citer en particulier le chlorhydrate d'aluminium sous forme activée ou non activée, l'aluminium chlorohydrex, le complexe aluminium chlorohydrex polyéthylèneglycol, le complexe aluminium chlorohydrex propylèneglycol, l'aluminium dichlorohydrate, le complexe aluminium dichlorohydrex polyéthylèneglycol, le complexe aluminium dichlorohydrex propylèneglycol, l'aluminium sesquichlorohydrate, le complexe aluminium sesquichlorohydrex polyéthylèneglycol, le complexe aluminium sesquichlorohydrex propylèneglycol, le sulfate d'aluminium tamponné par le lactate de sodium et d'aluminium.

Parmi les sels d'aluminium et de zirconium, on peut citer en particulier l'aluminium zirconium octachlorohydrate, l'aluminium zirconium pentachlorohydrate, l'aluminium zirconium tetrachlorohydrate, l'aluminium zirconium trichlorohydrate.

Les complexes d'hydroxychlorure de zirconium et d'hydroxychlorure d'aluminium avec un acide aminé sont généralement connus sous l'appellation ZAG (lorsque l'acide aminé est la glycine). Parmi ces produits on peut citer les complexes aluminium zirconium octachlorohydrex glycine, aluminium zirconium pentachlorohydrex glycine, aluminium zirconium tetrachlorohydrex glycine et aluminium zirconium trichlorohydrex glycine.

On utilisera plus particulièrement le chlorhydrate d'aluminium sous forme activée ou non activée.

Les sels d'aluminium anti-transpirants peuvent être présents dans la composition selon l'invention à raison d'environ 0,5 à 25% en poids par rapport au poids total de la composition.

Afin d'améliorer l'homogénéité du produit, on peut utiliser en plus un ou plusieurs agents de suspension qui sont choisis de préférence parmi les argiles montmorillonites modifiées hydrophobes comme les bentonites ou hectorites modifiées hydrophobes. On peut citer par exemple le produit Stearalkonium Bentonite (nom CTFA) (produit de réaction de la bentonite et de l'ammonium quaternaire chlorure de stéaralkonium) tel que le produit commercial vendu sous le nom TIXOGEL MP 250 par la société Sud Chemie Rheologicals, United Catalysts Inc ou le produit Disteardimonium Hectorite (nom CTFA) (produit de réaction de l'hectorite et du chlorure de distéaryldimonium) vendu sous le nom de Bentone 38 ou Bentone Gel par la société Elementis Specialities.

Les agents de suspension sont présents de préférence dans des quantités allant de 0,1 à 5% en poids et plus préférentiellement de 0.2 à 2% en poids par rapport au poids total de la composition.

Les compositions selon l'invention peuvent également contenir en plus au moins une charge.

Parmi les charges utilisables selon l'invention, on peut citer les poudres organiques. On entend dans la présente demande par « poudre organique », tout solide insoluble dans le milieu à température ambiante (25°C).

Comme poudres organiques qui peuvent être utilisées dans la composition de l'invention, on peut citer par exemple, les particules de polyamide et notamment celles vendues sous les dénominations ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les microsphères de polyméthacrylate de méthyle, commercialisées sous la dénomination MICROSPHERE M-100 par la société Matsumoto ou sous la dénomination COVABEAD LH85 par la société Wackherr ; les poudres de copolymère éthylène-acrylate, comme celles commercialisées sous la dénomination FLOBEADS par la société Sumitomo Seika Chemicals ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast sous les références 551 DE 12 (granulométrie d'environ 12 µm et masse volumique 40 kg/m3), 551 DE 20 (granulométrie d'environ 30 µm et masse volumique 65 kg/m3), 551 DE 50 (granulométrie d'environ 40 µm), ou les microsphères commercialisées sous la dénomination MICROPEARL F 80 ED par la société Matsumoto ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone, notamment TOSPEARL 240 ; les poudres d'aminoacides telles que la poudre de Lauroyllysine commercialisée sous la dénomination AMIHOPE LL-11 par la Société Ajinomoto ; les particules de microdispersion de cire, qui ont de préférence des dimensions moyennes inférieures à 1 µm et notamment allant de 0,02 µm à 1 µm, et qui sont constituées essentiellement d'une cire ou d'un mélange de cires, telles que les produits commercialisés sous la dénomination Aquacer par la société Byk Cera, et notamment : Aquacer 520 (mélange de cires synthétiques et naturelles), Aquacer 514 ou 513 (cire de polyéthylène), Aquacer 511 (cire polymérique), ou telles que les produits commercialisés sous la dénomination Jonwax 120 par la société Johnson Polymer (mélange de cires de polyéthylène et de paraffine) et sous la dénomination Ceraflour 961 par la société Byk Cera (cire de polyéthylène modifiée micronisée) ; et leurs mélanges.

La composition cosmétique selon l'invention peut comprendre en outre des adjuvants cosmétiques choisis parmi les cires, les adoucissants, les antioxydants, les opacifiants, les stabilisants, les agents hydratants, les vitamines, les parfums, des bactéricides, les conservateurs, les polymères, les parfums, les agents épaississants, des agents propulseurs ou tout autre ingrédient habituellement utilisé en cosmétique pour ce type d'application.

Bien entendu, l'homme de métier veillera à choisir ce ou ces éventuels composés complémentaires de manière telle que les propriétés avantageuses attachées intrinsèquement à la composition cosmétique conforme à l'invention ne soient pas, ou substantiellement pas, altérées par la ou les adjonctions envisagées.

Les cires peuvent être choisies parmi les cires animales, fossiles, végétales, minérales ou de synthèse. On peut citer notamment les cires d'abeille, les cires de Carnauba, de Candellila, de canne à sucre, du Japon, les ozokérites, la cire de Montan, les cires microcristallines, les paraffines, les cires et résines de silicone.

Les épaississants, de préférence non ioniques, peuvent être choisis parmi les gommes de guar et celluloses modifiées ou non modifiées telles que la gomme de guar hydroxypropylée, la cétylhydroxyéthylcellulose, les silices comme par exemple la Bentone Gel MIO vendue par la société NL INDUSTRIES ou la Veegum Ultra, vendue par la société POLYPLASTIC.

Les quantités de ces différents constituants pouvant être présents dans la composition cosmétique selon l'invention sont celles classiquement utilisées dans compositions déodorantes.

Les compositions selon l'invention peuvent également contenir également un ou plusieurs autres agents structurants ou gélifiants de la phase liquide organique non-miscible dans l'eau de la composition tels que les alcools gras solides linéaires et/ou les cires ; les acides gras ou leurs sels (acide stérique, stéarate de sodium, acide 12-hydroxystéarique) ; les dibenzylidene alditols (DBS) ; le lanostérol, les dérivés du N-acyl amino-acide ; les dérivés de di ou triacides carboxyliques comme les alkyl N, N'-dialkylsuccinamides (ie : dodécyl N, N'-dibutylsuccinamide) ;les organopolysiloxane élastomères tels que ceux décrits dans la demande WO97/44010.

La composition selon l'invention peut encore être pressurisée et être conditionnée dans un dispositif aérosol.

La présente invention a pour objet un dispositif aérosol constitué par :
(A) un récipient comprenant une composition déodorante telle que définie précédemment,
(B) au moins un agent propulseur et un moyen de distribution de la dite composition aérosol.

Les propulseurs généralement utilisés dans ce type de produits et bien connus de l'homme de l'art, sont comme par exemple le diméthyléther (DME) ; les hydrocarbures volatils tels que le n-butane, le propane, l'isobutane, et leurs mélanges, éventuellement avec au moins un hydrocarbure chloré et/ou fluoré; parmi ces derniers on peut citer les composés vendus par la société Dupont de Nemours sous les dénominations Fréon® et Dymel®, et en particulier le monofluorotrichlorométhane, le difluorodichlorométhane, le tétrafluorodichloroéthane et le 1,1-difluoroéthane vendu notamment sous la dénomination commerciale DYMEL 152 A par la société DUPONT. On peut également utiliser en tant qu'agent propulseur le gaz carbonique, le protoxyde d'azote, l'azote ou l'air comprimé.

La composition contenant le ou les actifs déodorants et le ou les agents propulseurs peuvent se trouver dans le même compartiment ou dans des compartiments différents dans le récipient aérosol. Selon l'invention, la concentration en agent propulseur varie généralement de 5 à 95% en poids pressurisée et plus préférentiellement de 50 à 85% en poids par rapport au poids total de la composition pressurisée.

Le moyen de distribution, qui forme une partie du dispositif aérosol, est généralement constitué par une valve de distribution commandée par une tête de distribution, elle même comprenant une buse par laquelle la composition aérosol est vaporisée. Le récipient contenant la composition pressurisée peut être opaque ou transparent. Il peut être en verre, en matériau polymérique ou en métal, recouvert éventuellement d'une couche de vernis protecteur.

La présente invention a également pour objet un procédé cosmétique pour traiter les odeurs axillaires humaines, consistant à appliquer sur la surface axillaire une quantité efficace d'une composition telle que définie ci-dessus.

Les exemples qui suivent servent à illustrer la présente invention. Les quantités des ingrédients sont exprimées en pourcentages en poids par rapport au poids total de la composition.

### Exemples 1 et 2 de solutions hydro-alcooliques de pidolate de zinc et stabilité au stockage après deux mois à l'étuve à 45°C

| **Ingrédients** | **Exemple 1** **(hors invention)** | **Exemple 2** **(invention)** |
|---|---|---|
| Ethanol à 96% volume (4% d'eau) | 20 | 20 |
| Pidolate de zinc | 0,5 | 0,5 |
| Acide salicylique | - | 0,055 |
| Eau | qsp 100 | Qsp 100 |
| **Aspect après 2 mois de conservation à 45 °C** | Liquide limpide avec précipités | Liquide limpide sans précipité |

### Exemples 3 à 5 d'aérosols alcooliques de salicylate de zinc et stabilité au stockage après deux mois à l'étuve à 45 °C

| **Ingrédients** | **Exemple 3** **(hors invention)** | **Exemple 4** **(invention)** | **Exemple 5** **(invention)** | **Exemple 6** **(hors invention)** |
|---|---|---|---|---|
| Ethanol à 96% volume (4% d'eau) | 42,9 | 42,9 | 44,4 | 44,5 |
| Salicylate de zinc | 0,60 | 0,47 | 0,50 | 0,47 |
| Acide salicylique | - | 0,13 | 0,10 | - |
| Parfum | 1,5 | 1,5 | - | - |
| Isobutane | 55 | 55 | 55 | 55 |
| Aspect après 2 mois de conservation à 45°C | Liquide limpide avec précipités | Liquide limpide sans précipité | Liquide limpide sans précipité | Liquide limpide avec précipités |

### Tests d'efficacité in-vivo de l'aérosol alcoolique de l'exemple 7 par rapport à un aérosol alcoolique à base de Triclosan

On effectue un test sensoriel sur un panel de 20 volontaires. On applique une seule fois sur chaque volontaire une composition par aisselle dans une quantité de 1.2 ± 0.05 g vaporisée à 15 cm de l'aisselle. Le déodorant est appliqué après essuyage de l'aisselle. Un panel d'experts (5 personnes) évalue sous les aisselles des volontaires :
(1) l'intensité de l'odeur de transpiration, 8 heures après application et donne une note de 1 à 9 (1 : odeur de transpiration imperceptible et 9 : odeur extrêmement forte)
(2) l'intensité du désagrément ou la valeur hédonique, 8 et 24 heures après application et donne une note de 1 à 9 (1: odeur extrêmement désagréable et 9 : extrêmement agréable).

### Formules testées : Aérosols alcooliques

| **Ingrédients** | **Exemple 7** | **Exemple 8** **(hors invention)** |
|---|---|---|
| Alcool éthylique 96 ° dénaturé | 44,4% | 44,8% |
| Salicylate de zinc | 0,47% | - |
| Acide salicylique | 0,12% | - |
| Triclosan | - | 0,15 |
| Isobutane | 55,0 | 55,0 |

Les résultats obtenus sont résumés dans la tableau suivant :

### Résultats 8 heures après l'application

| | **Intensité de l'odeur de transpiration** | | **Valeur hédonique de l'odeur** | |
|---|---|---|---|---|
| | Aisselle traitée par l'exemple 7 | Aisselle traitée par l'exemple 8 | Aisselle traitée par l'exemple 7 | Aisselle traitée par l'exemple 8 |
| **Moyenne** | 3,9 | 3,7 | 3,7 | 3,7 |
| **Ecart-type** | 1,8 | 1,3 | 1,1 | 1,0 |
| | % de variation | + 5% | % de variation | 0% |

### Résultats 24 heures après l'application

| | **Intensité de l'odeur de transpiration** | | **Valeur hédonique de l'odeur** | |
|---|---|---|---|---|
| | Aisselle traitée par l'exemple 7 | Aisselle traitée par l'exemple 8 | Aisselle traitée par l'exemple 7 | Aisselle traitée par l'exemple 8 |
| **Moyenne** | 4,1 | 4,3 | 3,6 | 3,5 |
| **Ecart-type** | 1,8 | 1,6 | 1,1 | 1,1 |
| | % de variation | - 5% | % de variation | + 4% |

La composition de l'exemple 7 selon l'invention comprenant l'association salicylate de zinc/acide salicylique selon l'invention présente une efficacité déodorante équivalente à celle de l'exemple 8 comprenant le Triclosan.

### MISE EN EVIDENCE DE L'ACTIVITE BACTERICIDE DE L'ASSOCIATION SALICYLATE DE ZINC/ACIDE SALICYLIQUE VIS-A-VIS DES MICRO-ORGANISMES IMPLIQUES DANS LES ODEURS AXILLAIRES

Le test décrit ici permet la détermination quantitative de l'activité bactéricide d'un actif (ie : salicylate de zinc/acide salicylique ou Triclosan) véhiculé dans un support approprié neutre vis-à-vis des bactéries testées. Les bactéries étudiées sont des germes en conditions optimales de croissance à savoir des germes du type Corynebacterium xerosis (Collection de l'Institut Pasteur n°5216) cultivés sur gélose Tryptocaséine soja en pente.

La veille du test, dans un pilulier, on dépose 32 g de bouillon Tryptocaséine soja et l'on met à incuber à 35°C. Le jour du test, on ajoute 4 g de la composition à tester et on homogénéise au Vortex.

Un témoin de croissance sans produit est préparé dans les mêmes conditions afin de vérifier que les germes sont dans des conditions de croissance favorables pendant toute la durée du test.

Pour la préparation de l'inoculum, cinq jours avant le début du test, on pratique un repiquage des deux souches bactériennes sur milieu approprié. On incube 5 jours à 35°C. Le jour du test, on lave la pente avec environ 9 ml de diluant: La suspension obtenue titre à 108 germes/ml (on effectue un dénombrement). On introduit 4 ml d'inoculum dans le pilulier, ce qui correspond à un taux de 107 bactéries par gramme de préparation. On place le pilulier dans un incubateur-agitateur (35°C - 200 RPM).

Après chaque temps de contact (2, 4, 6 et 24 heures), on homogénéise le contenu du pilulier au Vortex. On effectue des dilutions décimales. On dépose en surface de boîtes de Pétri gélosée (milieu Eugon LT 100). On incube les boîtes de Pétri 6 à 7 jours à l'étuve à 35°C.

On procède au comptage des colonies sur les boîtes contenant plus de 20 et moins de 200 colonies.

En ce qui concerne l'évaluation en tant qu'actif, les résultats sont exprimés, pour chaque micro-organisme testé, en nombre de Log de réduction au bout de 24 heures par rapport au placebo :

| | |
|---|---|
| résultats identiques : | nulle, |
| 1 Log de réduction : | faible, |
| 2 Log de réduction : | moyenne, |
| 3 Log de réduction : | bonne, |
| ≥ 4 Log de réduction : | excellente. |

On a réalisé les deux gels supports A et B suivants (quantités exprimées en pourcentages en poids):

| **Ingrédients** | **Support A** **(invention)** | **Support B** **(art antérieur)** |
|---|---|---|
| Triclosan | | 0,1% |
| Salicylate de zinc | 0.47% | |
| Acide salicylique | 0.12% | |
| Polyéthyleneglycol à 8 OE | 6% | 6% |
| Copolymère réticulé acide acrylique/acrylate d'alkyle en C10-C30 | 0.9% | 0.9% |
| Hydroxyde de sodium pur | qs (pH=7) | qs (pH=7) |
| Eau désionisée microbiologiquement propre | qsp 100% | qsp 100% |

On mesure l'activité bactéricide de chacune des formulations 9 et 10 vis à vis de la souche Corynebacterium xerosis et on l'a compare avec une formule sans actif.

Les résultats obtenus sont résumés dans le tableau suivant :

| **Actif testé** | **Efficacité à 24 heures par rapport au gel sans actif** |
|---|---|
| Souches | Corynebacterium xerosis |
| **Salicylate de zinc et acide salicylique** | Faible |
| **Triclosan** | Excellent |

L'association salicylate de zinc/acide salicylique a une activité antibactérienne faible sur les souches Corynebacterium Xerosis. Il a donc un spectre d'activité bactéricide sensiblement moins large que celui du Triclosan et respecte d'avantage la flore cutanée

### Exemples 9 et 10 de solutions hydroalcooliques à base de salicylate de zinc et d'acide carboxylique

| **Ingrédients** | **Exemple 9** **(hors invention)** | **Exemple 10** **(invention)** |
|---|---|---|
| Salicylate de zinc | 0,91 | 0,91 |
| Acide citrique | 0,05 | - |
| Acide salicylique | - | 0,05 |
| Ethanol à 96% volume (4% d'eau) | qsp 100 | qsp 100 |
| **Aspect immédiat après fabrication** | Formation de précipité blanc | Absence de précipité blanc |

## Revendications

1. Composition déodorante comprenant dans un milieu cosmétiquement acceptable :
a) au moins un sel hydrosoluble de zinc,
b) l'acide salicylique,
c) au moins un monoalcool en C₁-C₅ et
d) au moins 2% en poids d'eau par rapport au poids total de la composition.

2. Composition selon la revendication 1, où le ou les sels hydrosolubles de zinc sont choisis parmi le pidolate de zinc, le sulfate de zinc, le chlorure de zinc, le lactate de zinc, le gluconate de zinc, le phénolsulfonate de zinc, le salicylate de zinc et ses dérivés ou leurs mélanges.

3. Composition selon la revendication 2, où le salicylate de zinc et ses dérivés répondent à la structure suivante : 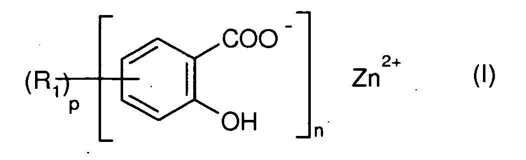 dans laquelle n = 2, p vaut 0, 1, 2 ou 3 ; R₁ désigne un alkyle en C₁-C₁₈ linéaire ou ramifié (par exemple méthyle, éthyle, n-propyle, isopropyle, n-butyle); un radical hydroxyalkyle en C₁-C₁₈, linéaire ou ramifié ; un atome d'halogène (par exemple Iode, Brome, Chlore) ; un radical acyle en C₂-C₁₈ (par exemple acétyle) ; un radical COR₂, OCOR₂ ou CONHR₂ où R₂ désigne un atome d'hydrogène ou un radical alkyle en C₁-C₁₈ linéaire ou ramifié.

4. Composition selon la revendication 3, où le sel hydrosoluble de zinc est le salicylate de zinc.

5. Composition selon l'une quelconque des revendications 1 à 4, où le ou les sels hydrosolubles de zinc sont présents dans des proportions pondérales, allant de de 0,1 à 5%, et mieux encore allant de 0,1 à 2%, par rapport au poids total de la composition.

6. Composition selon l'une quelconque des revendications 1 à 5, où l'acide salicylique est présent dans des proportions pondérales, allant de 0,01 à 0,5% et plus particulièrement de 0,05 à 0,2% en poids par rapport au poids total de la composition.

7. Composition selon l'une quelconque des revendications 1 à 6, où le ou les mono-alcools en C₁-C₅ sont choisis parmi le méthanol, l'éthanol, l'isopropanol ou leurs mélanges.

8. Composition selon l'une quelconque des revendications 1 à 7, où l'alcool en C₁-C₅ est l'éthanol.

9. Composition selon l'une quelconque des revendications 1 à 8, où le ou les mono-alcools en C₁-C₅ sont présents à des concentrations supérieures à 10% en poids par rapport au poids total de la composition et plus préférentiellement allant de 15 à 96 % en poids par rapport au poids total de la composition.

10. Composition selon l'une quelconque des revendications 1 à 9, où la quantité d'eau est de 2 à 85%, en poids et plus préférentiellement de 2 à 30% en poids par rapport au poids total de la composition.

11. Composition selon l'une quelconque des revendications 1 à 10, **caractérisée par le fait qu'**elle se présente sous forme de lotion distribuée en vaporisateur ou pompe aérosol , de crème distribuée en tube ou en grille; de gel distribué en roll-on ou en grille.

12. Composition selon l'une quelconque des revendications 1 à 11, **caractérisée par le fait qu'**elle se présente sous forme d'émulsion.

13. Composition selon l'une quelconque des revendications 1 à 12, **caractérisée par le fait qu'**elle contient en plus une phase liquide non-miscible dans l'eau.

14. Composition selon la revendication 13, où la phase liquide organique non-miscible dans l'eau contient une ou plusieurs huiles émollientes siliconée ou hydrocarbonée, volatiles ou non-volatiles.

15. Composition selon la revendication 14, où la ou les huiles émollientes sont présentes dans des quantités allant de 1 à 50% en poids et plus préférentiellement de 5 à 40% en poids par rapport au poids total de la composition.

16. Composition selon l'une quelconque des revendications 1 à 15, **caractérisée par le fait qu'**elle contient en plus un ou plusieurs actifs déodorants additionnels.

17. Composition selon l'une quelconque des revendications 1 à 16, **caractérisée par le fait qu'**elle contient en plus un ou plusieurs sels d'aluminium antitranspirants.

18. Composition selon l'une quelconque des revendications 1 à 17, **caractérisée par le fait qu'**elle contient en plus un ou plusieurs agents de suspension.

19. Composition selon l'une quelconque des revendications 1 à 18, **caractérisée par le fait qu'**elle contient en plus une ou plusieurs charges.

20. Composition selon l'une quelconque des revendications 1 à 19, **caractérisée par le fait qu'**elle contient en plus un ou plusieurs adjuvants cosmétiques choisis parmi les cires, les adoucissants, les antioxydants, les opacifiants, les stabilisants, les agents hydratants, les vitamines, les parfums, des bactéricides, les conservateurs, les polymères, les parfums, les agents épaississants, des agents propulseurs.

21. Composition selon l'une quelconque des revendications 13 à 20, **caractérisée par le fait qu'**elle contient en plus un ou plusieurs agents structurants ou gélifiants de la phase liquide organique non-miscible dans l'eau.

22. Dispositif aérosol constitué par :
(A) un récipient comprenant une composition déodorante telle que définie dans l'une quelconque des revendications précédentes ;
(B) au moins un agent propulseur et un moyen de distribution de la dite composition aérosol.

23. Utilisation de l'acide salicylique dans une composition déodorante comprenant dans un milieu cosmétiquement acceptable :
(a) au moins un sel hydrosoluble de zinc ,
(b) au moins 2% en poids d'eau par rapport au poids total de la composition,
(c) au moins un monoalcool en C₁-C₅ , comme agent stabilisant de la composition.

24. Procédé pour traiter les odeurs axillaires humaines, **caractérisé par le fait qu'**il consiste à appliquer une quantité efficace d'une composition telle que définie à l'une quelconque des revendications 1 à 21 sur la surface axillaire humaine.

## Claims

1. Deodorant composition comprising, in a cosmetically acceptable medium:
a) at least one water-soluble zinc salt,
b) salicylic acid,
c) at least one C₁-C₅ monoalcohol, and
d) at least 2% by weight of water, with respect to the total weight of the composition.

2. Composition according to Claim 1, where the water-soluble zinc salt or salts are chosen from zinc pidolate, zinc sulphate, zinc chloride, zinc lactate, zinc gluconate, zinc phenolsulphonate, zinc salicylate and its derivatives or their mixtures.

3. Composition according to Claim 2, where the zinc salicylate and its derivatives correspond to the following structure: 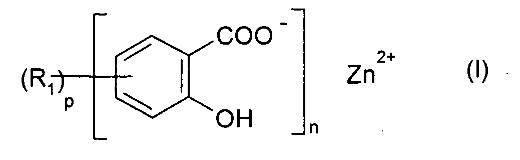 in which n = 2, p has the value 0, 1, 2 or 3 and R₁ denotes a linear or branched C₁-C₁₈ alkyl (for example methyl, ethyl, n-propyl, isopropyl or n-butyl), a linear or branched C₁-Cₗ₈ hydroxyalkyl radical, a halogen atom (for example, iodine, bromine, chlorine), a C₂-C₁₈ acyl radical (for example, acetyl) or a COR₂, OCOR₂ or CONHR₂ radical where R₂ denotes a hydrogen atom or a linear or branched C₁-C₁₈ alkyl radical.

4. Composition according to Claim 3, where the water-soluble zinc salt is zinc salicylate.

5. Composition according to any one of Claims 1 to 4, where the water-soluble zinc salt or salts are present in proportions by weight ranging from 0.1 to 5% and better still ranging from 0.1 to 2%, with respect to the total weight of the composition.

6. Composition according to any one of Claims 1 to 5, where the salicylic acid is present in proportions by weight ranging from 0.01 to 0.5% and more particularly from 0.05 to 0.2% by weight, with respect to the total weight of the composition..

7. Composition according to any one of Claims 1 to 6, where the C₁-C₅ monoalcohol or monoalcohols are chosen from methanol, ethanol, isopropanol or their mixtures.

8. Composition according to any one of Claims 1 to 7, where the C₁-C₅ alcohol is ethanol.

9. Composition according to any one of Claims 1 to 8, where the C₁-C₅ monoalcohol or monoalcohols are present at concentrations of greater than 10% by weight, with respect to the total weight of the composition, and more preferably ranging from 15 to 96% by weight, with respect to the total weight of the composition.

10. Composition according to any one of Claims 1 to 9, where the amount of water is from 2 to 85% by weight and more preferably from 2 to 30% by weight, with respect to the total weight of the composition.

11. Composition according to any one of Claims 1 to 10, **characterized in that** it is provided in the form of a lotion dispensed in a vaporizer or aerosol pump, of a cream dispensed in a tube or in a twist stick or of a gel dispensed in a roll-on device or in a twist stick.

12. Composition according to any one of Claims 1 to 11, **characterized in that** it is provided in the form of an emulsion.

13. Composition according to any one of Claims 1 to 12, **characterized in that** it additionally comprises a water-immiscible organic liquid phase.

14. Composition according to Claim 13, where the water-immiscible organic liquid phase comprises one or more volatile or nonvolatile silicone or hydrocarbon emollient oils.

15. Composition according to Claim 14, where the emollient oil or oils are present in amounts ranging from 1 to 50% by weight and more preferably from 5 to 40% by weight, with respect to the total weight of the composition.

16. Composition according to any one of Claims 1 to 15, **characterized in that** it additionally comprises one or more additional deodorant active principles.

17. Composition according to any one of Claims 1 to 16, **characterized in that** it additionally comprises one or more antiperspirant aluminium salts.

18. Composition according to any one of Claims 1 to 17, **characterized in that** it additionally comprises one or more suspending agents.

19. Composition according to any one of Claims 1 to 18, **characterized in that** it additionally comprises one or more fillers.

20. Composition according to any one of Claims 1 to 19, **characterized in that** it additionally comprises one or more cosmetic adjuvants chosen from waxes, softeners, antioxidants, opacifiers, stabilizers, moisturizing agents, vitamins, fragrances, bactericides, preservatives, polymers, thickening agents or propellants.

21. Composition according to any one of Claims 13 to. 20, **characterized in that** it additionally comprises one or more other structuring or gelling agents for the water-immiscible organic liquid phase.

22. Aerosol device, composed of:
(A) a container comprising a deodorant composition as defined in any one of the preceding claims;
(B) at least one propellant and a means for dispensing the said aerosol composition.

23. Use of salicylic acid in a deodorant composition comprising, in a cosmetically acceptable medium:
(a) at least one water-soluble zinc salt,
(b) at least 2% by weight of water, with respect to the total weight of the composition,
(c) at least one C₁-C₅ monoalcohol,
as stabilizing agent for the composition.

24. Method for treating human axillary odours, **characterized in that** it consists in applying an effective amount of a composition as defined in any one of Claims 1 to 21 to the human axillary surface.

## Patentansprüche

1. Desodorierende Zusammensetzung, die in einem kosmetisch akzeptablen Medium enthält:
a) mindestens ein wasserlösliches Zinksalz,
b) Salicylsäure,
c) mindestens einen einwertigen C₁-C₅-Alkohol und
d) mindestens 2 Gew.-% Wasser, bezogen auf das Gesamtgewicht der Zusammensetzung.

2. Zusammensetzung nach Anspruch 1, bei der das wasserlösliche Zinksalz oder die wasserlöslichen Zinksalze ausgewählt sind unter Zinkpidolat, Zinksulfat, Zinkchlorid, Zinklactat, Zinkgluconat, Zinkphenolsulfonat und Zinksalicylat sowie ihren Derivaten oder Gemischen.

3. Zusammensetzung nach Anspruch 2, bei der das Zinksalicylat und seine Derivate der nachstehenden Struktur entsprechen, 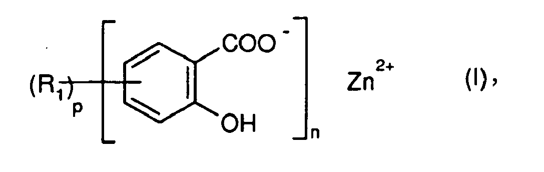 in der bedeuten:
n den Wert 2,
p 0, 1, 2 oder 3,
R₁ geradkettiges oder verzweigtes C₁-C₁₈-Alkyl (zum Beispiel Methyl, Ethyl, n-Propyl, Isopropyl, n-Butyl); geradkettiges oder verzweigtes C₁-C₁₈-Hydroxyalkyl; ein Halogenatom (zum Beispiel Iod, Brom, Chlor); C₂-C₁₈-Acyl (zum Beispiel Acetyl); eine Gruppe COR₂, OCOR₂ oder CONHR₂, worin R₂ ein Wasserstoffatom oder eine geradkettige oder verzweigte C₁-C₁₈-Alkylgruppe bezeichnet.

4. Zusammensetzung nach Anspruch 3, bei der das wasserlösliche Zinksalz Zinksalicylat ist.

5. Zusammensetzung nach einem der Ansprüche 1 bis 4, bei der das wasserlösliche Zinksalz oder die wasserlöslichen Zinksalze in Gewichtsanteilen von 0,1 bis 5 % und noch günstiger von 0,1 bis 2 % vorliegen, bezogen auf das Gesamtgewicht der Zusammensetzung.

6. Zusammensetzung nach einem der Ansprüche 1 bis 5, bei der die Salicylsäure in Gewichtsanteilen von 0,01 bis 5 Gew.-% und insbesondere von 0,05 bis 0,2 Gew.-% vorliegt, bezogen auf das Gesamtgewicht der Zusammensetzung.

7. Zusammensetzung nach einem der Ansprüche 1 bis 6, bei welcher der einwertige C₁-C₅-Alkohol oder die einwertigen C₁-C₅-Alkohole unter Methanol, Ethanol, Isopropanol oder ihren Gemischen ausgewählt sind.

8. Zusammensetzung nach einem der Ansprüche 1 bis 7, bei welcher der C₁-C₅-Alkohol Ethanol ist.

9. Zusammensetzung nach einem der Ansprüche 1 bis 8, bei welcher der einwertige C₁-C₅-Alkohol oder die einwertigen C₁-C₅-Alkohole in Konzentrationen von mehr als 10 Gew.-%, bezogen auf das Gesamtgewicht der Zusammensetzung, und noch bevorzugter in Konzentrationen von 15 bis 96 Gew.-% vorliegen, bezogen auf das Gesamtgewicht der Zusammensetzung.

10. Zusammensetzung nach einem der Ansprüche 1 bis 9, bei der die Menge an Wasser 2 bis 85 Gew.-% und noch bevorzugter 2 bis 30 Gew.-% beträgt, bezogen auf das Gesamtgewicht der Zusammensetzung.

11. Zusammensetzung nach einem der Ansprüche 1 bis 10, **dadurch gekennzeichnet, dass** sie vorliegt in Form einer als Zerstäuber oder Aerosolpumpe vertriebenen Lotion, einer in einer Tube oder einem Tiegel vertriebenen Creme oder eines als Roller oder im Tiegel vertriebenen Gels.

12. Zusammensetzung nach einem der Ansprüche 1 bis 11, **dadurch gekennzeichnet, dass** sie in Form einer Emulsion vorliegt.

13. Zusammensetzung nach einem der Ansprüche 1 bis 12, **dadurch gekennzeichnet, dass** sie ferner eine flüssige, nicht mit Wasser mischbare Phase enthält.

14. Zusammensetzung nach Anspruch 13, bei der die nicht mit Wasser mischbare flüssige organische Phase ein oder mehrere flüchtige oder nichtflüchtige Siliconöle oder Kohlenwasserstofföle enthält, die als Emollientien wirken.

15. Zusammensetzung nach Anspruch 14, bei der das oder die als Emollientien wirkenden Öle in Mengenanteilen von 1 bis 50. Gew.-% und noch bevorzugter von 5 bis 40 Gew.-% vorliegen, bezogen auf das Gesamtgewicht der Zusammensetzung.

16. Zusammensetzung nach einem der Ansprüche 1 bis 15, **dadurch gekennzeichnet, dass** sie ferner einen oder mehrere zusätzliche desodorierende Wirkstoffe enthält.

17. Zusammensetzung nach einem der Ansprüche 1 bis 16, **dadurch gekennzeichnet, dass** sie ferner ein oder mehrere Aluminiumsalze als Antitranspirantien enthält.

18. Zusammensetzung nach einem der Ansprüche 1 bis 7, **dadurch gekennzeichnet, dass** sie ferner ein oder mehrere Suspendiermittel enthält.

19. Zusammensetzung nach einem der Ansprüche 1 bis 18, **dadurch gekennzeichnet, dass** sie ferner einen oder mehrere Füllstoffe enthält.

20. Zusammensetzung nach einem der Ansprüche 1 bis 19, **dadurch gekennzeichnet, dass** sie ferner einen oder mehrere kosmetische Hilfsstoffe enthält, die ausgewählt sind unter Wachsen, reizlindernden Mitteln, Antioxidantien, Trübungsmitteln, Stabilisierungsmitteln, Hydratisierungsmitteln, Vitaminen, Parfums, bakteriziden Mitteln, Konservierungsmitteln, Polymeren, Parfums, Verdickungsmitteln und Treibmitteln. ,

21. Zusammensetzung nach einem der Ansprüche 13 bis 20, **dadurch gekennzeichnet, dass** sie ferner ein oder mehrere strukturbildende Mittel oder gelbildende Mittel der organischen, nicht mit Wasser mischbaren flüssigen Phase enthält.

22. Aerosolvorrichtung, die besteht aus:
(A) einem Behälter, der eine desodorierende Zusammensetzung nach einem der vorhergehenden Ansprüche enthält,
(B) mindestens einem Treibmittel und einer Einrichtung zur Abgabe der Aerosolzusammensetzung.

23. Verwendung von Salicylsäure in einer desodorierenden Zusammensetzung, die in einem kosmetisch akzeptablen Medium enthält:
a) mindestens ein wasserlösliches Zinksalz,
b) mindestens 2 Gew.-% Wasser, bezogen auf das Gesamtgewicht der Zusammensetzung,
c) mindestens einen einwertigen C₁-C₅-Alkohol
als Stabilisierungsmittel der Zusammensetzung

24. Verfahren zur Behandlung von menschlichem Achselgeruch, **dadurch gekennzeichnet, dass** es in der Anwendung einer wirksamen Menge einer Zusammensetzung, wie sie in einem der Ansprüche 1 bis 21 definiert ist, auf die Oberfläche der menschlichen Achsel besteht.
